# EUROPÄISCHE PATENTSCHRIFT

(11) **EP 1 192 958 B1**
(45) Veröffentlichungstag und Bekanntmachung des Hinweises auf die Patenterteilung: **07.05.2003**
(21) Anmeldenummer: 01118224.3
(22) Anmeldetag: 30.07.2001
(51) Int. Cl.: A61L 31/12, A61B 19/08, B32B 25/20

(54) **Mehrschichtige Textilware für chirurgische Abdeckungen und Bekleidungsstücke**
Multilayer textile product for surgical covering and clothing
Textiles stratifiés pour vêtements et tissus chirurgicaux

(30) Priorität: 28.08.2000 DE 10042198
(43) Veröffentlichungstag der Anmeldung: 03.04.2002
(73) Patentinhaber: Bernard, Axel, 72458 Albstadt-Ebingen (DE)
(72) Erfinder: Bernard, Axel, 72458 Albstadt-Ebingen (DE)
(74) Vertreter: Rüger, Barthelt & Abel Patentanwälte

(56) Entgegenhaltungen:
- EP-A- 0 507 760
- DE-A- 4 031 942
- FR-A- 2 737 106

## Beschreibung

Die Erfindung betrifft eine mehrschichtige Textilware für chirurgische Abdeckungen und Bekleidungsstücke und ein Verfahren zur Herstellung solcher Textilware.

Chirurgische Abdeckungen, sogenannte OP-Abdeckungen und chirurgische Bekleidungsstücke in Form von OP-Kitteln, Mänteln, Umhängen, Hosen, Hauben und dergleichen müssen einerseits gegenüber Luft, Flüssigkeiten, Keimen und Viren möglichst undurchlässig sein, sollen andererseits auf ihrer dem Patienten zugewandten Außenseite sich durch eine nur geringe Saugfähigkeit auszeichnen und müssen schließlich so beschaffen sein, dass sie im Falle der Wiederverwendung gewaschen und einwandfrei sterilisiert werden können ohne dabei Schaden zu leiden. Weil insbesondere die Lebensdauer wiederverwendbarer OP-Abdeckungen und OP-Bekleidungen wegen der hohen Beanspruchungen, denen sie beim Waschen und Sterilisieren ausgesetzt werden, begrenzt ist, werden in der Praxis in großem Umfang Einwegmaterialien für diese Zwecke verwendet. Dadurch ist nicht nur ein erheblicher wirtschaftlicher Aufwand erforderlich, sondern die Entsorgung der in Krankenanstalten anfallenden gebrauchten Einwegmaterialien stößt aus ökologischen Gründen auf zunehmende Bedenken.

Für wiederverwendbare OP-Abdeckungen und OP-Bekleidungsstücke gibt es Textilwaren in Form von Laminaten, die Beschichtungen und/oder Membranen auf der Basis von Polyurethan, Polytetrafluorethylen (PTFE) und modifiziertem Polyester/Polyamid aufweisen. Die Beschichtungen und Membranen sind mit Gestricken, Gewirken, Geweben und auch Vliesen, kurz mit textilen Flächengebilden im Transferverfahren beschichtet, direkt beschichtet oder aber unter Verwendung verschiedener Kleber im Punkt- oder Strichlaminierverfahren verklebt. Die Beschichtungen und/oder Membranen sind gegenüber Wasserdampf nicht oder teilweise mehr oder weniger durchlässig, während sie gegenüber Luft, Flüssigkeiten, wie Blut und Spülflüssigkeiten, Keimen und Viren als Barriere wirken.

Aus der DE 40 31 943 A1 ist ein sterilisierbares chirurgisches Tuch bekannt das als flüssigkeitsabsorbierende Außenschicht nicht gewebte Vliese auf Basis thermoplastischer Kunststoffe, eine damit haftfest verbundene flüssigkeitsundurchlässige Barriereschicht aus einer thermoplastischen Kunststofffolie und eine mit der Barriereschicht haftfest verbundene hautfreundliche, atmungsaktive, durchlässige Basisschicht aus einem Vlies auf Basis von thermoplastischen Kunststofffasern aufweist. Die Außenschicht, die Barriereschicht und die Basisschicht sind durch thermische Verfestigung infolge kurzzeitiger Erhitzung zumindest einer der Schichten bis auf Temperaturen im Erweichungsbereich des eingesetzten Kunststoffes miteinander verbunden. Dieses chirurgische Tuch verwendet für alle drei Schichten gleiche thermoplastische Kunststoffe; seine Machart beschränkt den Anwendungsbereich.

Nach Gebrauch werden OP-Abdeckungen und OP-Bekleidungsstücke bei ca 70°C mit desinfizierenden Chemikalien/Waschmitteln bei alkalischen PH-Werten gewaschen und in günstigen Fällen im letzten Spülbad abgesäubert, um einer Hydrolyse der Polyester(PES)Garne der textilen Schichten und der Beschichtungen und/oder Membranen entgegenzuwirken. Das Waschgut wird anschließend durch Schleudern oder Pressen entwässert, um die darauffolgenden Trockenzeiten zu verkürzen. Die Trocknung erfolgt sodann in Tumblern bei Temperaturen bis zu 210°C. Nach der Kontrolle auf Beschädigungen müssen, wiederum unter Temperatur- und Druckeinwirkung, Reparaturpatche auf beschädigte Stellen aufgebracht werden. Schließlich werden nach dem Falten, Legen und Zusammenbinden (Tapen) sogenannte Sets gepackt, die bei 134°C im fraktionierten Verfahren mit Dampf sterilisiert werden. Abgesehen von der sich durch diese Wasch-, Spül- und Trocknungsvorgänge, etc. ergebenden hohen Beanspruchungen werden die OP-Abdeckungen und OP-Bekleidungsstücke im Operationssaal selbst der Einwirkung diverser chemischer Flüssigkeiten und Gase ausgesetzt.

Die heute bekannten, zur Wiederverwendung bestimmten Textilwaren für OP-Abdeckungen und OP-Bekleidung sind diesen starken Beanspruchungen durchweg nicht in ausreichendem Maße gewachsen. Die Beschichtungen/Membranen sind in mehr oder weniger großem Maße temperaturempfindlich (Schmelzpunkt beginnend bei 150°C), nicht hydrolysebeständig, nicht genügend chemikalienbeständig und mechanisch empfindlich. Die eingesetzten Klebstoffe, die die Membranen-Beschichtungen mit den Textilien verbinden sind wiederum nicht temperatur- oder chemikalienresistent. Daher treten nach einer bestimmten Anzahl von sogenannten WTS-Zyklen (Waschen, Trocknen, Sterilisieren) Delaminationen auf. In der Praxis werden Artikel mit solchen Beschädigungen umgehend aus dem Verkehr gezogen, da eine Undichtigkeit unterstellt wird und sie deshalb für den medizinischen Weitergebrauch nicht mehr geeignet erscheinen.

Aufgabe der Erfindung ist es deshalb hier abzuhelfen und eine mehrschichtige Textilware für wiederverwendbare chirurgische Abdeckungen und Bekleidungsstücke zu schaffen, die sich bei einwandfreier Funktionalität durch eine hohe Wirtschaftlichkeit und dadurch auszeichnet, dass sie eine gegenüber bekannten Produkten wesentlich erhöhte Zahl von WTS-Zyklen durchlaufen kann ohne dabei beschädigt oder unbrauchbar zu werden.

Zur Lösung diese Aufgabe besteht die erfindungsgemäße mehrschichtige Textilware aus zwei Außenschichten aus textilen Flächengebilden und einer dazwischenliegenden dritten Schicht aus Silikonelastomeren, die mit den beiden benachbarten textilen Flächengebilden klebstoffrei verbunden ist.

Die dritte Schicht aus Silikonelastomeren besteht aus zu Elastomeren vernetzbaren Silikonkautschuken (Polyorganosiloxan). Die Vernetzung (Vulkanisation) kann durch Luftfeuchtigkeit oder Wärmezufuhr ausgelöst werden (RTV/HTV). Solche Produkte sind unter der eingetragenen Markenbezeichnung "Elastosil" der Wacker Chemie GmbH, Burghausen, DE auf dem Markt erhältlich.

In der bevorzugten Ausführungsform bestehen die Außenschichten aus Strick- oder Wirkware, doch ist die Erfindung darauf nicht beschränkt. Als textile Flächengebilde können für spezielle Einsatzzwecke auch Gewebe und Vliese oder Kombinationen derselben Verwendung finden. Zweckmäßigerweise weist eine der Außenschichten, bei einem medizinischen Abdecktuch die von dem Patienten entfernt liegende, distale Außenschicht, eine möglichst große Saugfähigkeit auf, während die andere proximale Außenschicht, die dem Patienten zugewandt ist, eine möglichst kleine Saugfähigkeit aufweisen soll. Die Außenschichten sind mit Vorteil aus Polyesterfasermaterial gearbeitet oder enthalten zumindest dieses Fasermaterial. Besonders zweckmäßig ist es, wenn in den gestrickten oder gewirkten textilen Außenschichten Endlos-Polyester-filamentgarne verarbeitet sind, wobei endlose Filamentgarne aus Polyester (PES) auch ausschließlich eingesetzt sein können. Dadurch ergeben sich positive Eigenschaften hinsichtlich einer geringen Partikelabgabe.

Die Bindung ist so gewählt, dass im Zusammenhang mit dem verwendeten Garn geringst mögliche Schrumpfwerte und eine möglichst kleine Dehnung in Längs- und Querrichtung erzielt werden.

Die stark saugende Wirkung der einen textilen Außenschicht kann erzielt werden einerseits durch die Verwendung von hochkapillaren, texturierten Polyestergarnen und andererseits durch die, gegebenenfalls zusätzliche, Verwendung von hydrofilisierenden Materialien bei der Veredelung oder Ausrüstung der Garne oder der Textilware.

Auf der dem Patienten zugewandten Seite einer OP-Abdeckung soll eine möglichst geringe Saugwirkung vorherrschen, um zu vermeiden, dass Blut und Flüssigkeiten aus einer Wunde gesaugt werden. Die Dochtwirkung der Nähte wird deshalb auf dieser Seite ebenfalls verringert. Um diese möglichst geringe Saugwirkung zu erreichen, werden in der entsprechenden Außenschicht des Textilmaterials keine texturierten Garne eingesetzt.

Die Herstellung der neuen Textilware für chirurgische Abdeckungen und Bekleidungsstücke kann an sich mit jedem geeigneten Verfahren geschehen. Besonders zweckmäßig ist jedoch das erfindungsgemäße Verfahren gemäß dem Patentanspruch 10.

Weiterbildungen der Textilware und dieses Verfahrens sind Gegenstand von Unteransprüchen.

Die neue Textilware ist frei von Klebstoffen und daher nicht der Gefahr einer Delamination ausgesetzt, wie sie von der Verminderung der Haftfestigkeit an Klebestellen bei bekannten Produkten ausgehen kann. Das Silikonelastomere der dritten Schicht ist bis über 200°C temperaturbeständig, d.h. einem Wert, der weit oberhalb aller in den Aufbereitungsprozessen bei der Wiederverwendung vorkommenden Temperaturen liegt. Außerdem sind Silikonelastomere gegenüber den meisten Chemikalien resistent.

Aus dem neuen Textilmaterial bestehende, wiederverwendbare OP-Abdeckungen und OP-Bekleidungen können, wie die praktische Erfahrung gezeigt hat, ohne Beschädigung mehr als 60 bis 100 WTS-Zyklen durchlaufen. Sie kommen hinsichtlich ihrer Lebensdauer damit erstmals in einen Bereich, in dem sie im Vergleich zu Einwegmaterialien wirtschaftlich sind. Der Benutzer erhält ein wiederstandsfähiges Produkt, das bei der Verwendung allen Anforderungen in hervorragendem Maße gerecht wird und dennoch eine vielfache Wiederverwendung ohne Beschädigung gestattet.

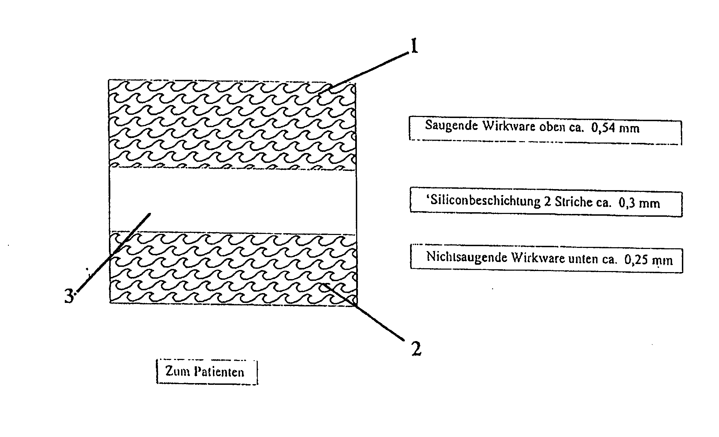

In der Zeichnung ist ein Ausführungsbeispiel des Gegenstandes der Erfindung dargestellt. Die schematische Skizze zeigt einen Querschnitt durch eine Textilware gemäß der Erfindung.

Die in der Skizze im Querschnitt schematisch dargestellte erfindungsgemäße mehrschichtige Textilware ist ein 3-Schichtlaminat und dient zur Herstellung von chirurgischen Abdeckungen und Bekleidungsstücken. Sie besteht aus zwei Außenschichten 1, 2 aus textilen Flächengebilden und einer dazwischenliegenden dritten Schicht 3 aus Silikonelastomeren, die mit den beiden benachbarten textilen Flächengebilden der Außenschichten 1, 2 klebstofffrei verbunden ist.

Bei Verwendung für ein chirurgisches Abdecktuch (OP-Abdeckung) ist die Außenschicht 1 distal, d.h. vom Patienten abgewandt angeordnet, während die andere Außenschicht 2, proximal, d.h. dem Patienten benachbart liegt.

Die Außenschicht 1 oder Oberschicht besteht bei dem beschriebenen Ausführungsbeispiel aus einer Wirkware hoher Saugfähigkeit und weist eine Dicke von ca. 0,54 mm auf.

Sie ist ausschließlich aus endlosen Polyester-Filamentgarnen gearbeitet und enthält ein hochkapillares, texturiertes Polyestergarn zur Erzielung einer starken Saugwirkung. Außerdem sind die Garne und/oder die Wirkware mit hydrofilisierenden Produkten veredelt. Die Verwendung von ausschließlich endlosen Filamentgarnen ergibt positive Eigenschaften hinsichtlich einer geringen Partikelabgabe.

| Garn und Bindung der saugenden Schicht 1: | | |
|---|---|---|
| | Bindung | Garn |
| Legeschiene 1 (hinten): | 10/12// Trikot | PES 33/18 glatt rund halbmatt |
| Legeschiene 2: (vorne) : | 23/10// Tuch | PES 50/40 texturiert, profiliert glänzend |

Die gewählte Bindung ergibt im Zusammenhang mit den eingesetzten Garnen geringstmögliche Schrumpfwerte, wie sie für den Einsatz notwendig sind.

Die gegenüberliegende Außenschicht 2 oder Unterschicht zeichnet sich durch eine möglichst geringe Saugfähigkeit aus. Sie weist bei dem beschriebenen Ausführungsbeispiel eine Dicke von ca. 0,25 mm auf und besteht aus einer Wirkware, die ausschließlich aus endlosen Polyesterfilamentgarnen gearbeitet ist. Die Wirkware enthält keine texturierten Garne, um so eine möglichst geringe Saugwirkung zu erzielen.

Die Bindung ist so gewählt, dass Dehnungen in Längsund Querrichtung auf ein Mindestmaß reduziert sind. Außerdem werden durch die gewählte Bindung die an sich schon gegebenen guten Schrumpfwerte noch verstärkt.

| Garn und Bindung der nicht saugenden Außenschicht 2: | | |
|---|---|---|
| | Bindung | Garn |
| Legeschiene 1 (hinten): | 34/10// Satin | PES 33/18 glatt rund halbmatt |
| Legeschiene 2: (vorne): | 10/12// Trikot | PES 55/30 glatt rund halbmatt |

Die dritte Schicht 3 aus Silikonelastomeren weist eine Dicke von ca. 0,3 mm auf. Sie ist lückenlos und porenfrei und für Luft, Wasserdampf, Flüssigkeiten, sowie Keime und Viren undurchlässig. Sie besteht aus zu Elastomeren vernetzbaren Silikonkautschuken. Die Vernetzung kann durch Luftfeuchtigkeit oder Wärmezufuhr ausgelöst werden.

Zur Herstellung der neuen Textilware kann an sich jedes geeignete Beschichtungsverfahren verwendet werden, das es erlaubt eine direkte klebstofffreie Verbindung zwischen den Textilschichten und der Silikonelastomeren-Schicht herzustellen.

Besonders zweckmäßig ist die Verwendung folgenden Verfahrens:

In einem ersten Schritt wird ein Träger, bspw. in Gestalt eines geeigneten Papiers, dünn mit nicht ausvulkanisierter Silikonelastomeren-Paste beschichtet. In die noch nasse Paste wird die dünnere und leichtere Wirkware der Außenschicht 2 einkaschiert, worauf das Ganze getrocknet wird, damit die erste Silikonelastomeren-Schicht ausvulkanisiert (ausreagiert).

Das auf diese Weise erzeugte 2-lagige Laminat besitzt die notwendige gleichmäßige Dicke und bestimmt die Eigenschaft der sicheren Flüssigkeitsbarriere.

In einem zweiten Schritt wird sodann das so hergestellte 2-lagige Laminat von dem Träger abgenommen und als Vorlage für die weitere Verarbeitung genommen. Dazu wird auf der Silikonelastomeren-Seite dieses Laminats in einem zweiten Strich eine zweite pastöse, nicht ausvulkanisierte Silikonelastomeren-Schicht aufgebracht. Anschließend wird die dickere, schwerere Wirkware der Außenschicht 1 in die noch nasse zweite Silikonelastomeren-Schicht einkaschiert, worauf diese ebenfalls ausvulkanisiert (ausreagiert) lassen wird.

Der zweite Strich dient somit hauptsächlich als Verbindungsmittel für die Wirkware der Außenschicht 1.

Nach dem Ausvulkanisieren der zweiten Silikonelastomeren-Schicht liegt die fertige neue Textilware in Gestalt eines 3-Lagenlaminats vor.

Für bestimmte Anwendungsfälle wäre es auch denkbar in ähnlicher Weise Textilware für chirurgische Abdeckungen und Bekleidungsstücke in Gestalt von Mehrlagenlaminaten aufzubauen, die mehr als drei Schichten enthalten.

## Patentansprüche

1. Mehrschichtige Textilware für chirurgische Abdeckungen und Bekleidungsstücke, bestehend aus zwei Außenschichten (1,2) aus textilen Flächengebilden und einer dazwischenliegenden dritten Schicht (3) aus Silikonelastomeren, die mit den beiden benachbarten textilen Flächengebilden (1,2) klebstofffrei verbunden ist.

2. Textilware nach Anspruch 1, **dadurch gekennzeichnet, dass** die Außenschichten (1,2) aus Strick- oder Wirkware bestehen.

3. Textilware nach Anspruch 1 oder 2, **dadurch gekennzeichnet, dass** eine der Außenschichten (1) eine möglichst große und die andere Außenschicht (2) eine möglichst kleine Saugfähigkeit aufweisen.

4. Textilware, nach einem der vorhergehenden Ansprüche, **dadurch gekennzeichnet, dass** die Außenschichten (1,2) aus Polyesterfasermaterial gearbeitet sind oder dieses enthalten.

5. Textilware nach Anspruch 4, **dadurch gekennzeichnet, dass** in den Außenschichten (1,2) endlose Polyester-Filamentgarnen verarbeitet sind.

6. Textilware nach einem der Ansprüchen 3 bis 5, **dadurch gekennzeichnet, dass** die Außenschicht (1) großer Saug fähigkeit texturiertes Polyestergarn enthält.

7. Textilware nach Anspruch 6, **dadurch gekennzeichnet, dass** wenigstens eine Außenschicht unter Verwendung von hydrofilisierenden Materialien veredelt ist.

8. Textilware nach Anspruch 4 oder 5, **dadurch gekennzeichnet, dass** die Außenschicht (2) mit geringer Saugfähigkeit zumindest teilweise aus glattem Polyestergarn gearbeitet ist.

9. Textilware nach einem der Ansprüche 2 bis 8, **dadurch gekennzeichnet, dass** die Außenschichten (1,2) mit einer die Dehnungsfähigkeit in Längs- und Querrichtung auf ein Mindestmaß reduzierenden Bindung gearbeitet sind.

10. Verfahren zur Herstellung einer mehrschichtigen Textilware nach einem der vorhergehenden Ansprüche, bei dem
- in einem ersten Schritt nicht ausvulkanisiertes, pastöses Silikonelastomeres in dünner Schicht auf einen Träger aufgebracht, sodann ein textiles Flächengebilde in die noch nasse Paste einkaschiert und anschließend die Silikonelastomeren-Schicht unter Ausbildung eines Laminates ausvulkanisiert (ausreagiert) wird und
- in einem zweiten Schritt das so hergestellte Laminat von dem Träger abgenommen wird, auf der
Silikonelastomeren-Seite des Laminates eine zweite, nicht ausvulkanisierte pastöse Silikonelastomerenschicht aufgebracht wird, sodann in die noch nasse, zweite Silikonelastomeren-Schicht ein textiles Flächengebilde einkaschiert und anschließend die zweite Silikonelastomeren-Schicht ausvulkanisiert (ausreagiert) wird.

11. Verfahren nach Anspruch 10, **dadurch gekennzeichnet, dass** bei beiden Schritten eine Strick- oder Wirkware in die jeweilige Silikonelastomeren-Schicht einkaschiert wird.

12. Verfahren nach Anspruch 11, **dadurch gekennzeichnet, dass** zwei verschieden schwere Wirkwaren verwendet werden und dass in dem ersten Schritt die dünnere und leichtere Wirkware und in dem zweiten Schritt die dickere und schwerere Wirkware in die jeweilige Silikonelastomeren-Schicht einkaschiert wird.

13. Verwendung der Textilware nach einem der Ansprüche 1 bis 9 zur Herstellung chirurgischer Abdeckungen und Bekleidungsstücke.

## Claims

1. Multilayer textile product for surgical coverings and articles of clothing, comprising two outer layers (1, 2) made of textile fabrics and an interposed third layer (3) made of silicone elastomers, which is joined to the two adjacent textile fabrics (1, 2) to be free from adhesive.

2. Textile product according to Claim 1, **characterised in that** the outer layers (1, 2) are made of knitted or hosiery products.

3. Textile product according to Claim 1 or 2, **characterised in that** one of the outer layers (1) has as high an absorbency as possible and the other outer layer (2) has as low an absorbency as possible.

4. Textile product according to one of the preceding claims, **characterised in that** the outer layers (1, 2) are worked from polyester fibre material or contain this.

5. Textile product according to Claim 4, **characterised in that** continuous polyester filament yarns are processed into the outer layers (1, 2).

6. Textile product according to one of Claims 3 to 5, **characterised in that** the outer layer (1) with higher absorbency contains textured polyester yarn.

7. Textile product according to Claim 6, **characterised in that** at least one outer layer is finished using hydrophilising materials.

8. Textile product according to Claim 4 or 5, **characterised in that** the outer layer (2) with lower absorbency is worked at least partially from smooth polyester yarn.

9. Textile product according to one of Claims 2 to 8, **characterised in that** outer layers (1, 2) are worked with a structure which reduces the stretching property in longitudinal and transverse direction to a minimum.

10. Process for the production of a multilayer textile product according to one of the preceding claims, in which
- in a first step paste-like silicone elastomer material that has not fully cured is applied onto a support material, a textile fabric is then bonded into the still wet paste and the silicone elastomer layer is subsequently fully cured (fully cross-linked) to form a laminate, and
- in a second step the thus produced laminate is removed from the support material, a second paste-like silicone elastomer layer that has not fully cured is applied on the silicone elastomer side of the laminate, a textile fabric is then bonded into the still wet second silicone elastomer layer and the second silicone elastomer layer is subsequently fully cured (fully cross-linked) .

11. Process according to Claim 10, **characterised in that** a knitted or hosiery product is bonded into the respective silicone elastomer layer in both steps.

12. Process according to Claim 11, **characterised in that** two hosiery products of different weights are used, and that in the first step the thinner and lighter hosiery product is bonded into the respective silicone elastomer layer and in the second step the thicker and heavier hosiery product is bonded into the respective silicone elastomer layer.

13. Use of the textile product according to one of Claims 1 to 9 for the production of surgical coverings and articles of clothing.

## Revendications

1. Textile stratifié pour tissus et vêtements chirurgicaux, constitué de deux couches extérieures (1,2) formées d'un textile plat et d'une troisième couche (3) intermédiaire en silicone élastomère, qui est liée aux textiles plats voisins (1,2) sans adhésif.

2. Textile selon la revendication 1, **caractérisé par le fait que** les couches extérieures (1,2) sont en tricot ou en tissu à mailles.

3. Textile selon la revendication 1 ou 2, **caractérisé par le fait qu'**une des couches extérieures (1) a un pouvoir absorbant le plus élevé possible et que l'autre couche extérieure (2) a un pouvoir absorbant le plus faible possible.

4. Textile selon l'une des revendications précédentes, **caractérisé par le fait que** les couches extérieures (1,2) sont en un matériau fibreux en polyester ou contiennent un tel matériau.

5. Textile selon la revendication 4, **caractérisé par le fait que** les couches extérieures (1,2) contiennent des filaments en polyester sans fin.

6. Textile selon l'une des revendications 3 à 5, **caractérisé par le fait que** la couche extérieure (1) fortement absorbante contient des fils en polyester texturés.

7. Textile selon la revendication 6, **caractérisé par le fait qu'**au moins une des couches extérieures est affinée par la présence de matériaux hydrophiles.

8. Textile selon la revendication 4 ou 5, **caractérisé par le fait que** la couche extérieure (2) faiblement absorbante est constituée, au moins partiellement, de fils de polyester lisses.

9. Textile selon l'une des revendications 2 à 8, **caractérisé par le fait que** la liaison des couches extérieures (1,2) est faite de manière à réduire au maximum l'extensibilité dans le sens de la longueur et de la largeur.

10. Procédé de fabrication d'un textile stratifié selon l'une des revendications précédentes, dans lequel
- on applique, dans une première étape, une silicone élastomère pâteuse non vulcanisée en une mince couche sur un support, on applique ensuite un textile plat dans la pâte encore humide et l'on soumet ensuite la couche de silicone élastomère à une vulcanisation complète aboutissant à la formation d'un stratifié, et
- on retire, dans une deuxième étape, le stratifié ainsi préparé du support, on applique sur la face du stratifié formée par la couche de silicone élastomère une deuxième couche en silicone élastomère pâteuse non vulcanisée, on applique sur la deuxième couche de silicone élastomère, encore humide, un deuxième textile plat et l'on soumet ensuite la deuxième couche de silicone élastomère à une vulcanisation complète.

11. Procédé selon la revendication 10, **caractérisé par le fait que** l'on applique lors des deux étapes, du tricot ou du tissu à mailles sur les couches en silicone élastomère.

12. Procédé selon la revendication 11, **caractérisé par le fait que** l'on utilise deux tissus à mailles de masses différentes et que l'on applique dans la première étape le tissu à mailles plus mince et plus léger, et dans la deuxième étape le tissu à mailles plus épais et plus lourd sur la couche de silicone élastomère correspondante.

13. Utilisation du textile selon l'une des revendications 1 à 9 pour la fabrication de tissus et de vêtements chirurgicaux.
